Europäisches Patentamt

European Patent Office

Office européen des brevets

(19)

(11) Publication number : **0 478 685 B1**

(12) # EUROPEAN PATENT SPECIFICATION

(45) Date of publication of patent specification :
28.07.93 Bulletin 93/30

(51) Int. Cl.⁵ : **C12N 11/08, C12P 7/62**

(21) Application number : **90910608.0**

(22) Date of filing : **15.06.90**

(86) International application number :
**PCT/DK90/00152**

(87) International publication number :
**WO 90/15868 27.12.90 Gazette 90/29**

(54) **IMMOBILIZED LIPASE PREPARATION AND USE THEREOF FOR ESTER SYNTHESIS.**

(30) Priority : **21.06.89 DK 3061/89**

(43) Date of publication of application :
**08.04.92 Bulletin 92/15**

(45) Publication of the grant of the patent :
**28.07.93 Bulletin 93/30**

(84) Designated Contracting States :
**AT BE CH DE DK ES FR GB IT LI NL SE**

(56) References cited :
**EP-A- 0 232 933**
**EP-A- 0 320 132**
**EP-A- 0 322 213**
**WO-A-88/02775**
**WO-A-89/02916**

(56) References cited :
**Chemical Abstracts, vol. 111, no.1, 3 July 1989,
(Columbus, Ohio, US), Heldt-Hansen, Hans Pe-
ter et al "A new immobilized positional non
specific lipase for fat modification and ester
synthesis", see page 301, abstract 3085c**

(73) Proprietor : **NOVO NORDISK A/S**
**Novo Allé**
**DK-2880 Bagsvaerd (DK)**

(72) Inventor : **PEDERSEN, Sven**
**Emil Reesensvej 9**
**DK-2820 Gentofte (DK)**
Inventor : **EIGTVED, Peter**
**Parcelvej 42 A**
**DK-2840 Holte (DK)**

(74) Representative : **Bach, Niels**
**c/o Novo Nordisk A/S Patent Dept. Novo Allé**
**DK-2880 Bagsvaerd (DK)**

## Description

The technical area to which this invention belongs comprises immobilized lipase preparations and us thereof for ester synthesis.

One of the immobilization methods for lipases comprises adsorption from an aqueous solution to a particulate, porous carrier with affinity for the lipase. The selection of a carrier is a delicate problem: in the first place the carrier has to exhibit a high selective affinity to the special lipase in question in the aqueous solution; in the second place the thus produced immobilized lipase preparation has to exhibit a high activity and stability during use conditions, whereby use conditions may be ester synthesis or ester hydrolysis; in the third place the carrier has to be relatively cheap, if the immobilized lipase preparation should be used on an industrial scale; and in the fourth place the carrier has to be non-toxic, and be able to be food approved.

*Candida antarctica* lipase A and *Candida antarctica* lipase B are known lipases, vide e.g. EP 287.634, and also, it is described in EP 287.634 that these lipases can be immobilized by adsorption on anion exchange resins of the Duolite® type. However, for ester synthesis purposes these prior art preparations were unsatisfactory, as they exhibited rather low ester synthesis activities.

Thus the purpose of the invention is the provision of an immobilized lipase preparation prepared by adsorption of the lipase on a carrier from an aqueous solution of the lipase in which the lipase is one or both of the lipases selected from *Candida antarctica* lipase A and *Candida antarctica* lipase B, whereby the immobilized lipase preparation should exhibit a higher ester synthesis activity under use conditions than the ester synthesis activity under use conditions of the prior art immobilized lipase preparations of this kind.

Now, surprisingly it has been found that it is possible to fulfil the purpose of the invention by selection of a specific carrier category chosen among a vast number of carrier categories, an by selection of a special pretreatment of the chosen carrier.

Thus, the immobilized lipase preparation for ester synthesis according to the invention is characterized by the fact that the lipase is one or both of the lipases selected from the group consisting of Candida antarctica lipase A and *Candida antarctica* lipase B, and that the immobilization is performed by adsorption from aqueous solution on a particulate porous aliphatic olefinic polymer, said polymer being pretreated with a polar, organic, water miscible solvent, in which the polymer is insoluble and which does not inactivate the lipase.

Surprisingly it has been found that the ester synthesis activity of the immobilized lipase preparation according to the invention is much higher than the corresponding prior art ester synthesis activity, which will be documented in detail in a later part of the specification.

Reference is made to EP 232.933, which describes the special category of carriers used in the invention together with some other categories of carriers. It is indicated in the EP patent No. 232.933 that lipase originating from *Candida cylindracea* can be used. No indication is made of the possibility of using *Candida antarctica* lipase A and *Candida antarctica* lipase B. In regard to structure and properties the lipase from *Candida cylindracea* is completely different from the lipases from *Candida antarctica* A and/or B. Furthermore, the EP patent is concerned exclusively with hydrolysis of fats, i.e. with reaction systems with high water concentrations, whereas the invention is concerned with ester synthesis, i.e. with systems with low water concentrations to extreme low water concentrations.

In WO published patent application no. 89/02916 it is described that *Candida lipase* immobilized on adsorbing resins of the acrylate type ("Lewatit") exhibit a higher interesterification activity than *Candida lipase* immobilized on adsorbing resins of the phenol type and weak anion exchange resins. It has been found, however, that the immobilized lipase preparation according to the invention, especially the immobilized lipase preparation according to the invention with *Candida antarctica* lipase B, at low water concentrations (0-1% w/w) exhibit a much higher interesterification activity than the interesterification activity of the prior art preparation immobilized on Lewatit, vide Example 6 in the following. In relation to interesterification processes it is highly advantageous to work with low water concentrations due to the corresponding low formulation of byproducts.

In this specification with claims use is made of the following terminology.

The term "porous" is intended to mean that the carrier particles exhibit pores. The smaller the pores the better, as smaller pores is equivalent to bigger surface areas. The pores, however, should preferably not be smaller than 100 Å in diameter, as the lipase molecules tend to be too large to enter such small pores. Thus, in accordance with the definition in "Colloid and Interface Chemistry" Vold and Vold, 1983, Addison-Wesley Publishing Company, Inc., page 101 the preferred pore structure in the immobilized lipase preparation is to be classified as macropores and/or mesopores.

The term "ester synthesis" encompasses all kinds of ester synthesis, e.g. a reaction between an alcohol and an acid, a reaction between an ester and an alcohol (alcoholysis), a reaction between two esters (transesterification), or a reaction between an ester and an acid (acidolysis). The term "interesterification" covers transesterification, acidolysis, and alcoholysis. The term "ester synthesis" also covers glycolipid synthesis, i.e.

a reaction between the alcohol glucose and a fatty acid.

The LU, which is the lipase unit used for evaluation of the activity in relation to fat hydrolysis, is described in the publication AF 95/5-GB, which is obtainable on request from Novo Nordisk A/S, Novo Allé, DK-2880 Bagsvaerd, Denmark.

The BIU, which is the lipase unit used for evaluation of the interesterification activity, is described in the publication AF 206-2, which is obtainable on request from Novo Nordisk A/S, Novo Allé, DK-2880 Bagsvaerd, Denmark.

The PLU, which is the lipase unit used for evaluation of the ester synthesis activity, is determined as follows:

Principle:

The activity is measured approximately as initial rate of n-propyl laurate formation.

Lipase preparation:

an immobilized preparation of a *Candida antarctica* lipase A and/or *Candida antarctica* lipase B is hydrated to 10% w/w (overnight).

Substrate:

250 mg enzyme preparation (dry substance)
40 mmole lauric acid (8.01 g)
40 mmole n-propanol (2.40 g)

Reaction:

60°C, 20 minutes, shaking in 20 ml glass vials (as in relation to BIU-assay).

Calculation of activity:

The acid value (AV) of the substrate is determined before and after reaction Degree of ester formation is calculated and initial activity is approximated:

$$\text{Activity} = \frac{40 \text{ mmole x (Degree of ester formation)}}{0.33 \text{ h x } 0.250 \text{ g}} \text{ [mmole/h/g]}$$

$$\text{Degree of ester formation} = \frac{AV_o - AV}{AV_o}$$

In a preferred embodiment of the immobilized lipase preparation according to the invention the carrier is polypropylene. Practical experiments have shown that this olefinic polymer is very satisfactory as a carrier.

In a preferred embodiment of the immobilized lipase preparation according to the invention more than 90% of the particles exhibit particle sizes between 100 and 1000 $\mu$m. If the particle size is below 100 $\mu$m, the pressure loss in a column will tend to be too large, and if the particle size is above 1000 $\mu$m, the diffusion inhibition tends to be too high.

Also, the invention comprises a use for ester synthesis of the immobilized lipase preparation according to the invention.

In a preferred embodiment of the use according to the invention the lipase is *Candida antarctica* lipase A, and the ester synthesis is an interesterification. Immobilization yield and interesterfication activity have been found to be most satisfactory.

In a preferred embodiment of the use according to the invention the lipase is *Candida antarctica* lipase B, and the ester synthesis is a reaction between an alcohol and an acid. Immobilization yield and ester synthesis activity have been found to be most satisfactory.

In a preferred embodiment of the use according to the invention the lipase is *Candida antarctica* lipase B, and the ester synthesis is an interesterification. Interesterification activity at low water concentration has been found to be most satisfactory.

The invention will be illustrated by means of the following examples. In these examples three preparations of Candida antarctica are investigated, i.e. *Candida antarctica* lipase A (abbreviated LA), *Candida antartica* lipase B (abbreviated LB) and the mixed preparation (abbreviated L(A+ B)). LA and LB are described and char-

EP 0 478 685 B1

acterized in EP 287.634.

The carriers used according to the invention and the control carriers belong to the types ACCUREL® and DUOLITE®, respectively. ACCUREL, which is a particulate polypropylene, is described in AKZO, Fibres and Polymers division, Accurel systems, datasheet, obtainable from Enka AG, Postfach D-8753 Obernburg, West Germany.

Duolite is a trademark from Rohm & Haas for anion and cation exchange resins and resinous adsorbent agents.

**EXAMPLE 1**

Comparison

A suspension of 4.0 g (dry matter) of Duolite ES-568N was adjusted to pH 7, and 0.80 g of LA (222,800 LU/g) dissolved in deionized water (total weight of LA solution 12 g) was added. pH was readjusted to 7, and the suspension was stirred for 2 hours at room temperature. The product was filtered and rinsed with 60 ml of deionized water and 60 ml of cold acetone. The product was vacuum dried at 40°C.
Immobilization yield = 34% (calculated from LU determinations)
Load = 15,700 LU/g of dry matter
BIU/g = 7; PLU/g = 0

**EXAMPLE 2**

According to the invention

4.0 g (dry matter) of Accurel EP 100 is slurried in 96% ethanol. The excess of ethanol is sucked away (residual ethanol 9 g). 0.80 g of LA (222,800 LU/g) dissolved in deionized water (total weight of LA solution is 20 g) is added. pH is adjusted to 7, and the suspension is stirred for 2 hours at room temperature. The product is filtered and rinsed with 60 ml of deionized water. The product is vacuum dried at 40°C.
Immobilization yield = 96% (calculated from LU determinations)
Load = 38,000 LU/g of dry matter
BIU/g = 56; PLU/g = 0

It appears from Examples 1 and 2 that the immobilized lipase preparation according to the invention is highly superior in comparison to the corresponding prior art preparation, both in regard to load and to interesterification activity.

**EXAMPLE 3**

According to the invention

10 g (dry matter) of Accurel EP 100 is slurried in 96% ethanol. The excess of ethanol is sucked away (residual ethanol 23 g). 34.7 g of LB (14,400 LU/g) dissolved in deionized water (total weight of LB solution is 50 g) is added. pH is adjusted to 7, and the suspension is stirred overnight for a total of 18 hours. The product is filtered and rinsed with 100 ml of deionized water. The product is vacuum dried at 40°C.
Immobilization yield = 100% (calculated from LU determinations)
Load = 48,200 LU/g of dry matter
BIU/g approx. 0; PLU/g = 370

A similar experiment, in which Duolite was used instead of Accurel, showed a much lower immobilization yield and a much lower ester synthesis activity.

**EXAMPLE 4**

Comparison

A suspension of 4.0 g (dry matter) of Duolite ES-568N was adjusted to pH 7, and 1.87 g of L(A+B) (85,600 LU/g) dissolved in deionized water was added (total weight of the L(A+B) solution was 12 g). pH was readjusted to 7, and the suspension was stirred overnight for a total of 17 hours. The product was filtered and rinsed with 60 ml of deionized water. The product was vacuum dried at 40°C.
Immobilization yield = 13% (calculated on LU)

4

Load = 5,400 LU/g of dry matter
BIU/g = 8; PLU/g = 0.2

## EXAMPLE 5

According to the invention

1 g (dry matter) of Accurel EP 100 was mixed with 10 ml of 96% ethanol. Stirring was carried out for 10 minutes, followed by vacuum filtration. 0.4 g of L(A+B) (142,000 LU/g) dissolved in 5 ml deionized water was added. pH was adjusted to 6.5, and the suspension was stirred overnight. The product was filtered and rinsed with 50 ml of deionized water. The product was air dried at room temperature.
Immobilization yield = 98% (calculated from LU determinations)
Load = 55,900 LU/g dry matter
BIU/g = 61; PLU/g = 346

It appears from Examples 4 and 5, that the immobilized lipase preparation according to the invention is highly superior to the corresponding prior art preparation, both in regard to the immobilization yield, the load and to the ester synthesis activity and the interesterification activity.

## EXAMPLE 6

According to the invention

This example describes the use of LA and LB for interesterification.
Procedures are as in Example 3, except for the following modifications and results:
4.43 g of LB (112,900 LU/g) to 4.0 g Accurel EP 100
Immobilization yield = 100%
Load = 45,200 LU/g of dry matter
PLU/g = 370 (same as in Example 3)
25 g Accurel EP 100
5.61 g of LA (222,800 LU/g)
Immobilization yield = 99%
Load = 44,700 LU/g of dry matter
BIU/g = 59

The interesterification activity was measured at catalyst hydrations from 0-10%:

| Hydration (% $H_2HO$) | 0-1 | 2 | 5 | 10 |
|---|---|---|---|---|
| Interesterification activity (BIU/g): | | | | |
| Immobilized LB | 14 | 10 | 0-1 | 0-1 |
| Immobilized LA | | 12 | | 59 |

It is remarkable that interesterification with LB is possible only at very low hydration levels.

## EXAMPLE 7

According to the invention

This example shows immobilization of LB on polyethylene powder, Accurel EP 400, with ethanol and isopropanol as wetting agents.

Variations from Example 3 are:

0.338 g of LB (360,000 LU/g) on 4.1 g Accurel EP 400 / ethanol
Immobilization yield = 30%
Load = 9,200 LU/g of dry matter

PLU/g = 369
0.341 g of LB (360,000 LU/g) on 4.1 g Accurel EP 400 / iso-PrOH
Immobilization yield = 22%
Load = 6,800 LU/g of dry matter
PLU/g = 294

## Claims

1.  Immobilized lipase preparation for ester synthesis, wherein the lipase is one or both of the lipases selected from the group consisting of *Candida antarctica* lipase A and *Candida antarctica* lipase B, and wherein the immobilization is performed by adsorption from aqueous solution on a particulate porous aliphatic olefinic polymer, said polymer being pretreated with a polar, organic, water miscible solvent, in which the polymer is insoluble and which does not inactivate the lipase.

2.  Immobilized lipase preparation according to Claim 1, wherein the polymer is a polypropylene.

3.  Immobilized lipase preparation according to Claim 1 or 2, wherein more than 90% of the particles exhibit particle sizes between 100 and 1000 µm.

4.  Use of the immobilized lipase preparation according to Claim 1 - 3 for ester synthesis.

5.  Use according to Claim 4, wherein the lipase is *Candida antarctica* lipase A, and the ester synthesis is an interesterification.

6.  Use according to Claim 4, wherein the lipase is *Candida antarctica* lipase B, and the ester synthesis is a reaction between an alcohol and an acid.

7.  Use according to Claim 4, wherein the lipase is *Candida antarctica* lipase B, and the ester synthesis is an interesterification.

## Patentansprüche

1.  Immobilisierte Lipasezubereitung zur Estersynthese, wobei die Lipase eine oder beide der aus der Gruppe, die aus Lipase A aus Candida antarctica und Lipase B aus Candida antarctica besteht, ausgewählten Lipasen ist und wobei die Immobilisierung durch Adsorption aus wäßriger Lösung auf einem teilchenförmigen, porösen, aliphatischen, olefinischen Polymer durchgeführt wird, wobei besagtes Polymer mit einem polaren, organischen, wassermischbaren Lösungsmittel vorbehandelt ist, in dem das Polymer unlöslich ist und das die Lipase nicht inaktiviert.

2.  Immobilisierte Lipasezubereitung nach Anspruch 1, wobei das Polymer ein Polypropylen ist.

3.  Immobilisierte Lipasezubereitung nach Anspruch 1 oder 2, wobei mehr als 90 % der Teilchen Teilchengrößen zwischen 100 und 1000 µm zeigen.

4.  Verwendung der immobilisierten Lipasezubereitung nach Anspruch 1-3 zur Estersynthese.

5.  Verwendung nach Anspruch 4, wobei die Lipase Lipase A aus Candida antarctica ist und die Estersynthese eine Umesterung ist.

6.  Verwendung nach Anspruch 4, wobei die Lipase Lipase B aus Candida antarctica ist und die Estersynthese eine Reaktion zwischen einem Alkohol und einer Säure ist.

7.  Verwendung nach Anspruch 4, wobei die Lipase Lipase B aus Candida antarctica ist und die Estersynthese eine Umesterung ist.

**Revendications**

1. Préparation de lipase immobilisée pour la synthèse d'esters, dans laquelle la lipase est une ou deux des lipases choisies dans le groupe consistant en lipase de candida antarctica A et en lipase de candida antarctica B et dans laquelle l'immobilisation s'effectue par adsorption à partir d'une solution aqueuse sur un polymère oléfinique aliphatique en particules poreux, ledit polymère étant prétraité à l'aide d'un solvant organique polaire miscible à l'eau dans lequel le polymère est insoluble et qui n'inactive pas la lipase.

2. Préparation de lipase immobilisée selon la revendication 1, dans laquelle le polymère est un polypropylène.

3. Préparation de lipase immobilisée selon la revendication 1 ou 2, dans laquelle plus de 90% des particules possèdent des tailles de particule entre 100 et 100 µm.

4. Utilisation de la préparation de lipase immobilisée selon les revendications 1 à 3 pour la synthèse d'esters.

5. Utilisation selon la revendication 4, dans laquelle la lipase est la lipase de candida antarctica A et la synthèse d'esters est une interestérification.

6. Utilisation selon la revendication 4, dans laquelle la lipase est la lipase de candida antarctica B et la synthèse d'esters est une réaction entre un alcool et un acide.

7. Utilisation selon la revendication 4, dans laquelle la lipase est la lipase de candida antarctica B et la synthèse d'esters est une interestérification.